(19) Europäisches Patentamt
European Patent Office
Office européen des brevets

(11) Veröffentlichungsnummer : **0 116 848**
**B1**

# (12) EUROPÄISCHE PATENTSCHRIFT

(45) Veröffentlichungstag der Patentschrift : **28.06.89**

(51) Int. Cl.⁴ : **A 61 K 7/06**

(21) Anmeldenummer : **84100384.1**

(22) Anmeldetag : **16.01.84**

## (54) Haarbehandlungsmittel.

(30) Priorität : **24.01.83 DE 3302210**

(43) Veröffentlichungstag der Anmeldung : **29.08.84 Patentblatt 84/35**

(45) Bekanntmachung des Hinweises auf die Patenterteilung : **28.06.89 Patentblatt 89/26**

(84) Benannte Vertragsstaaten : **AT BE CH DE FR GB IT LI LU NL SE**

(56) Entgegenhaltungen :
**DE–A– 1 295 138**
**FR–A– 1 393 604**
**US–A– 3 671 644**
**CHEMICAL ABSTRACTS, Band 96, Nr. 8, Februar 1982, Seite 354, Nr. 57590d, Columbus, Ohio, US; & JP - A - 81 139 412 (SHISEIDO CO. LTD.) 30-10-1981**
**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Ploog, Uwe, Dr.**
**Haydnweg 6**
**D-5657 Haan (DE)**
Erfinder : **Busch, Peter, Dr.**
**Gottfried-August-Bürger-Strasse 10**
**D-4006 Erkrath-Unterbach (DE)**
Erfinder : **Thiele, Klaus**
**Rügenweg 5**
**D-4018 Langenfeld (DE)**
Erfinder : **Höffkes, Horst, Dr.**
**Carlo-Schmid-Strasse 113**
**D-4000 Düsseldorf-Hellerhof (DE)**
Erfinder : **Giede, Karl**
**Schlehenweg 12**
**D-4010 Hilden (DE)**

**EP 0 116 848 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Gegenstand der Erfindung sind kosmetische untenstehend im einzelnen gekennzeichnete Haarbehandlungsmittel mit einem die Naßkämmbarkeit des Haares verbessernden Zusatz.

Das Haupthaar weist nach dem Waschen mit Shampoos auf Basis von synthetischen oberflächenaktiven Stoffen oft einen kosmetisch unbefriedigenden Zustand auf: Es fühlt sich stumpf an und ist im nassen Zustand nur schwer zu kämmen. Nach dem Trocknen neigt das gewaschene Haar zur statischen Aufladung, wodurch das Kämmen erschwert und der Sitz des gekämmten Haares gestört wird.

Es ist bekannt, nach dem Waschen oder Schamponieren des Haares konditionierende Präparate auf das Haar einwirken zu lassen. Dabei handelt es sich meist um gelförmige, flüssige oder emulsionsförmige Lotionen mit einem Gehalt an kationaktiven grenzflächenaktiven Stoffen. Es ist auch bekannt, üblichen Shampoos bestimmte Substanzen beizufügen, um gleichzeitig mit der Haarwäsche einen gewissen konditionierenden Effekt zu erzielen. Solche Substanzen sind z. B. wasserlösliche Proteine, Proteinabbauprodukte oder polykationische Polymere, z. B. kationische Cellulosederivate. Nachteilig bei den kationaktiven oberflächenaktiven Stoffen ist deren mangelhafte Verträglichkeit mit anionischen Tensiden und deren oft nicht befriedigende Schleimhautverträglichkeit.

Die polykationischen Polymeren wirken der statischen Aufladung des trockenen Haares nicht entgegen, in vielen Fällen wird die Aufladbarkeit des Haares sogar erhöht. Andererseits führt die starke Adsorption dieser kationischen Polymeren an die Keratinfaser, insbesondere bei mehrmaliger Anwendung zu einer Akkumulation der Polymeren auf dem Haar, welches dadurch « belastet » wird und Elastizität, Sitz und Fülle verliert.

Es bestand daher ein Bedürfnis an Haarpflegemitteln, welche die Naßkämmbarkeit des Haares verbessernde Zusätze enthalten und die geschilderten Nachteile nicht oder in geringerem Umfang aufweisen.

Aus DE-A-12 95 138 waren bereits Haarwaschmittelzusammensetzungen bekannt, die zur Verbesserung der Kämmbarkeit bestimmt Phosphonoverbindungen enthalten. In FR-A-1 393 604 werden Haarbehandlungsmittel beschrieben, die bestimmte Diphosphonate zur Verringerung der oxidativen Haarschädigung durch Bleich- und Haarfärbebehandlungen enthalten. Aus US-A-3671644 waren antiseptische Zubereitungen bekannt, die Phosphonate mit 2 oder 3 Phosphonatgruppen enthalten. In Chem. Abstr. 96, (1982) No. 57590 d sind haarfestigende Zubereitungen beschrieben, die neben razemischem Cystein komplexbildende Verbindungen, darunter 1-Hydroxyethan-1,1-diphosphonsäure enthalten.

Es wurde gefunden, daß die Naßkämmbarkeit des Haares durch Haarbehandlungsmittel deutlich verbessert wird, die als Zusatz bestimmte oberflächenaktive Phosphonsäuren enthalten. Diese Zusätze weisen die Nachteile der bekannten, die Naßkämmbarkeit verbessernden Zusätze nicht auf.

Gegenstand der Erfindung sind daher Haarbehandlungsmittel mit einem die Naßkämmbarkeit des Haares verbessernden Zusatz, dadurch gekennzeichnet, daß als Zusatz wenigstens eine oberflächenaktive Phosphonsäure der allgemeinen Formel (I)

$$R^1—CH_2—PO_3H_2$$

in der $R^1$ eine lineare Alkylgruppe mit 7-13 C-Atomen ist oder ein Salz davon enthalten ist.

Die als Zusätze zu den erfindungsgemäßen Haarbehandlungsmitteln geeigneten oberflächenaktiven Phosphonsäuren sind bekannte Verbindungen oder nach literaturbekannten Verfahren herstellbar. Zu ihnen gehören z. B. die Alkan-1-phosphonsäuren, die gemäß US-A-2 957 931 durch radikalische Anlagerung von Estern der phosphorigen Säure an Olefine und nachfolgende Verseifung hergestellt werden können.

Die am besten geeigneten Phosphonsäuren sind Octan-1-phosphonsäure, Decan-1-phosphonsäure und Dodecan-1-phosphonsäure. Die genannten Verbindungen können den Haarbehandlungsmitteln in Form der freien Säuren oder als Salze, bevorzugt als wasserlösliche Salze, zum Beispiel als Alkalisalze oder Ammoniumsalze zugesetzt werden. Die erfindungsgemäßen Haarbehandlungsmittel verbessern aufgrund des Zusatzes an oberflächenaktiven Phosphonsäuren oder deren Salzen die Naßkämmbarkeit des Haares. Sie reduzieren gleichzeitig die statische Aufladbarkeit des Haares, führen aber nicht zu einer Akkumulation der avivierenden Komponenten auf der Haaroberfläche mit den bekannten, damit verbundenen, unerwünschten Effekten.

Die Beständigkeit in alkalischen Rezepturen sowie gegenüber oxidierenden und reduzierenden Rezepturbestandteilen zeichnet sie gegenüber den meisten, die Naßkämmbarkeit verbessernden Zusätzen aus. Es ist daher möglich, mit diesen Zusätzen auch Haarfärbemittel, Blondiermittel und Dauerwellmittel auszustatten.

Die oberflächenaktiven Phosphonsäuren entfalten ihre Wirksamkeit schon in sehr geringen Anwendungskonzentrationen. Die erfindungsgemäßen Haarbehandlungsmittel können 0,05 bis 10 Gew.-% dieser Zusätze enthalten, eine Menge von 0,1 bis 2,0 Gew.-% der oberflächenaktiven Phosphonsäuren ist aber in den meisten Fällen für eine befriedigende Wirkung ausreichend.

Ein weiterer Vorteil der oberflächenaktiven Phosphonsäuren und deren Salzen ist die ausgezeichnete Verträglichkeit mit anionischen Tensiden, wie sie für viele haarkosmetische Zubereitungen, z. B. für Shampoos, Haarfärbemittel, Blondiermittel üblich sind.

Als anionische Tenside werden in erfindungsgemäßen Haarbehandlungsmitteln Alkylsulfate und/oder Alkylpolyglykolethersulfate mit 10-18 C-Atomen in der Alkylgruppe und bis zu 12 Polyglykolethergruppen und/oder Alkylpolyglykolether-sulfobernsteinsäuremonoester mit 10-16 C-Atomen in der Alkylgruppe und 2-6 Glykolethergruppen eingesetzt. Weitere geeignete Aniontenside für die Herstellung erfindungsgemäßer Haarbehandlungsmittel sind primäre und sekundäre lineare Alkansulfonate mit 10-18 C-Atomen, Alkensulfonate und Hydroxyalkansulfonate, wie sie bei der Sulfonierung von Olefinen mit 10-18 C-Atomen erhalten werden, Fettsäurealkylolamid- und Fettsäurealkylolamidpolyglykolether-sulfate, sulfatierte Fettsäuremonoglyceride, Alkylpolyglykolethercarboxylate mit 8-18 C-Atomen in der Alkylkette und 2-6 Glykolethergruppen, Acylsarkosine, Acyltauride und Acylisothionate mit 8-18 C-Atomen in der Acylgruppe.

Die genannten anionischen Tenside können in Form der Alkali-, Ammonium ; Alkanolammoniumsalze, die Alkylsulfate und Alkylpolyglykolethersulfate auch in Form der Magnesiumsalze vorliegen. Ihre Menge beträgt üblicherweise 2-50 Gew.-% des Haarbehandlungsmittels.

Eine besonders ausgeprägte Senkung des Kämmwiderstandes wurde bei Haarbehandlungsmitteln gefunden, welche die oberflächenaktiven Phosphonsäuren und als anionische Tenside eine Mischung aus einem Alkylpolyglykolethersulfat-Alkalisalz mit 10-16 C-Atomen in der Alkylgruppe und 2-4 Glykolethergruppen und einem Alkylpolyglykolethersulfobernsteinsäuremonoester-Alkalisalz mit 10-16 C-Atomen in der Alkylgruppe und 2-4 Glykolethergruppen enthalten.

Weiterhin können in den erfindungsgemäßen Haarbehandlungsmitteln auch andere oberflächenaktive Stoffe, z. B. nichtionogene, zwitterionische und amphotere Tenside und/oder sonst übliche Hilfs- und Zusatzstoffe wie Fettalkohole, wasserlösliche nichtionogene oder anionische Polymere, z. B. Celluloseether, Glykole, Salze, Puffersubstanzen, Konservierungsmittel, Farbstoffe, Duftstoffe, haarkosmetische Wirkstoffe, wie z. B. Antischuppenwirkstoffe, Sebostatika, Vitamine, Kräuterextrakte sowie Reduktionsmittel für die Haarverformung, Oxidationsmittel für die Fixierung der Haarverformung oder für die Haarbleichung sowie Oxidationsfarbstoffvorprodukte für die Färbung der Haare enthalten. Unter Verwendung der genannten Hilfs- und Zusatzmittel lassen sich die erfindungsgemäßen Haarbehandlungsmittel in der Form von Shampoos, Haarnachbehandlungsmittel, Haarkurpräparate, Dauerwellmittel, Dauerwellfixierung, Haarfärbemittel oder Haarbleichmittel formulieren. Die nachfolgenden Beispiele sollen den Gegenstand der Erfindung näher erläutern.

## Beispiele

1. Prüfung der Naßkämmbarkeit der Haare NK [%]

Die Wirkung der Zusätze von oberflächenaktiven Phosphonsäuren und deren Salzen auf die Naßkämmbarkeit der Haare wurde nach folgender Methode geprüft :

0,8 Gramm schwere, ca. 11 cm lange braune, europäische, durch einmalige Blondierung und Kaltwelle definiert vorbehandelte Haarsträhnen wurden mit einer Lösung von 2,0 Gew.-% der oberflächenaktiven Phosphonsäure in einer wässrigen Tensidlösung 5 Minuten lang bei 30 °C behandelt und anschließend in lauwarmem Wasser (30 °C) gründlich ausgespült und überschüssiges Wasser abgestreift. Sodann erfolgte die Messung des Kämmwiderstandes, d. h. der Kraft, die erforderlich war, um einen Kamm durch eine Haarsträhne zu ziehen. Zur Verringerung des Meßfehlers wurde die Bestimmung 15-fach mit jedem der zu prüfenden Produkte durchgeführt und der Mittelwert der Arbeitsintegrale gebildet. Die Messung erfolgte in einer Zug-Prüfmaschine des Typs 1402 der Fa. Zwick (Einsingen über Ulm/Donau).

Das mittlere Arbeitsintegral wurde auf das ohne Wirkstoff durch Behandlung mit der jeweiligen Tensidlösung erhaltene Arbeitsintegral (Blindwert) bezogen und zeigte so die Naßkämmbarkeitsverbesserung (oder Verschlechterung) :

$$NK\,[\%] = \frac{\text{Arbeitsintegral Wirkstoff}}{\text{Arbeitsintegral Blindwert}} \cdot 100$$

Werte unter 100 % bedeuten demnach eine Verbesserung, Werte über 100 % eine Verschlechterung der Naßkämmbarkeit der Haare.

Die Ergebnisse für die erfindungsgemäß in Haarbehandlungsmitteln einzusetzenden oberflächenaktive Phosphonate Octan- und Decan-1-phosphonsäure sowie Vergleichsverbindungen zeigt Tabelle I.

(Siehe Tabellen Seite 4 ff.)

Tabelle I

| oberflächenaktives Phosphonat | Tensid | Gew.% Tensid in $H_2O$ | NK [%] |
|---|---|---|---|
| Hexan-1-phosphonsäure (Vergleich) | Fettalkohol $C_{12-14}$ + 2EO-sulfat, Na-Salz | 14 | 78 % |
| Octan-1-phosphonsäure (erfindungsgemäß) | Fettalkohol $C_{12-14}$ + 2EO-sulfat, Na-Salz | 14 | 16 % |
| Decan-1-phosphonsäure (erfindungsgemäß) | Fettalkohol $C_{12-14}$ + 2EO-sulfat, Na-Salz | 14 | 20 % |
| Octadecan-1-phosphonsäure (Vergleich) | Fettalkohol $C_{12-14}$ + 2EO-sulfat, Na-Salz | 14 | 48 % |
| 1-Aminodecan-1,1-diphosphonsäure (Vergleich) | Fettalkohol $C_{12-14}$ + 2 EO-sulfat, Na-Salz | 14 | 66 % |

Tabelle I (Fortsetzung)

| oberflächenaktives Phosphonat | Tensid | Gew.% Tensid in $H_2O$ | NK [%] |
|---|---|---|---|
| Octan-1-phosphonsäure (erfindungsgemäß) | Laurylsulfat-triethanolammonium-Salz | 14 | 31 % |
| Octan-1-phosphonsäure (erfindungsgemäß) | Fettalkohol $C_{12-14}$ + 2EO-sulfat, Na-Salz<br>N-Hydroxyethyl-N-kokosalkylamido-ethylglycin | 11<br>3 | 41 % |
| Octan-1-phosphonsäure (erfindungsgemäß) | Fettalkohol $C_{12-14}$ + 2EO-sulfat, Na-Salz<br>Kokosalkylamidopropyl-dimethyl-glycin | 11<br>3 | 56 % |
| Octan-1-phosphonsäure (erfindungsgemäß) | Fettalkohol $C_{12-14}$ + 2 EO-sulfat, Na-Salz<br>Fettalkohol $C_{12-14}$ + 2 EO-sulfobernsteinsäuremonoester, Na-Salz | 11<br>3 | 9 % |
| 1-Hydroxyoctan-1.1-diphosphonsäure (Vergleich) | Fettalkohol $C_{12/14}$ + 2 EO-sulfat, Na-Salz | 14 | 64 % |

2. Rezepturen für erfindungsgemäße Haarpflegemittel

2.1 Shampoos

2.1.1 Avivageshampoo mit leichter Pflegewirkung

| | |
|---|---|
| Fettalkohol-$C_{12-14}$+ 2EO-sulfat, Na-Salz (28 %ig) (Texapon(R)N25) | 40 Gew.% |
| Kokosalkylamidopropyl-dimethylglycin (30 %ig) (Dehyton(R)K) | 10 Gew.% |
| Decan-1-phosphonsäure, Na-Salz | 0,1 Gew.% |
| Wasser, Farbstoffe, Duftstoffe, Konservierungsmittel | ad 100 Gew.% |

2.1.2 Shampoo für strapaziertes Haar

| | |
|---|---|
| Fettalkohol-$C_{12-14}$ + 2EO-sulfat, Na-Salz (28 %ig) | 40 Gew.% |
| Fettalkohol-$C_{12-14}$ + 2EO-sulfobernsteinsäuremonoester, Na-Salz (30 %ig) | 10 Gew.% |
| Octan-1-phosphonsäure, Na-Salz | 1,0 Gew.% |
| Wasser, Farbstoffe, Duftstoffe, Konservierungsmittel | ad 100 Gew.% |

Das mit diesen Shampoos gewaschene Haar zeigt gute Kämmbarkeit im nassen Zustand und keine elektrostatische Aufladung beim Kämmen nach dem Trocknen.

2.2 Haarnachbehandlungsmittel

2.2.1 Saures, abspülbares Haargel

| | |
|---|---|
| Octan-1-phosphonsäure | 5,0 Gew.% |
| Hydroxyethylcellulose | 0,8 Gew.% |
| Wasser, Duftstoffe, Farbstoffe, Konservierungsmittel | ad 100 Gew.% |

2.2.2 Emulsionsförmige, saure Haarspülung

| | |
|---|---|
| Cetyl-stearylalkohol | 1,5 Gew.% |
| Stearinsäuremono-diglycerid | 1,5 Gew.% |
| Cetyl-stearylalkoholpoly (20EO)-glykolether | 3,0 Gew.% |
| Nonylphenolpoly (6,5 EO) glykolether | 2,0 Gew.% |
| Octan-1-phosphonsäure | 2,0 Gew.% |
| Wasser, Farbstoffe, Duftstoffe, Konservierungsmittel | ad 100 Gew.% |

Die Nachspülmittel verleihen dem nassen und dem trockenen Haar eine bessere Kämmbarkeit und einen angenehmen Griff. Beim Kämmen des trockenen Haares mit einem Hartgummikamm tritt keine elektrostatische Aufladung ein.

**Patentansprüche**

1. Haarbehandlungsmittel mit einem die Naßkämmbarkeit des Haares verbessernden Zusatz, dadurch gekennzeichnet, daß als Zusatz wenigstens eine oberflächenaktive Phosphonsäure der allgemeinen Formel (I)

$$R^1—CH_2—PO_3H_2$$

in der $R^1$ eine lineare Alkylgruppe mit 7-13 C-Atomen ist oder ein Salz davon enthalten ist.

2. Haarbehandlungsmittel nach Anspruch 1, dadurch gekennzeichnet, daß Octan-1-phosphonsäure, Decan-1-phosphonsäure oder Dodecan-1-phosphonsäure oder ein Salz davon enthalten ist.

3. Haarbehandlungsmittel gemäß Anspruch 1 und 2 in Form eines Shampoos, dadurch gekennzeichnet, daß die oberflächenaktiven Phosphonsäuren oder deren wasserlösliche Salze in einer Menge von 0,05-10 Gew.-% sowie anionische Tenside in einer Menge von 2-50 Gew.-% des Mittels enthalten sind.

4. Haarbehandlungsmittel gemäß Anspruch 3, dadurch gekennzeichnet, daß die oberflächenaktiven Phosphonsäuren oder deren wasserlösliche Salze in einer Menge von 0,1-2,0 Gew.-% sowie als anionische Tenside eine Mischung aus einem Alkylpolyglykolethersulfat-Alkalisalz mit 10-16 C-Atomen in der Alkylgruppe und 2-4 Glykolethergruppen und einem Alkylpolyglykolether-sulfobersteinsäuremonoester-Alkalisalz mit 10-16 C-Atomen in der Alkylgruppe und 2-4 Glykolethergruppen in einer Menge von 2-50 Gew.-% des Haarbehandlungsmittels enthalten sind.

## Claims

1. Hair treatment preparations containing an additive for improving wet combability, characterized in that they contain at least one surface-active phosphonic acid corresponding to general formula (I)

$$R^1—CH_2—PO_3H_2$$

in which $R^1$ is a linear alkyl group containing 7 to 13 C atoms, or a salt thereof as the additive.

2. Hair treatment preparations as claimed in claim 1, characterized in that they contain octane-1-phosphonic acid, decane-1-phosphonic acid or dodecane-1-phosphonic acid or a salt thereof.

3. Hair treatment preparations as claimed in claims 1 and 2 in the form of a shampoo, characterized in that the surface-active phosphonic acids or their water-soluble salts are present in a quantity of from 0.05 to 10 % by weight and anionic surfactants are present in a quantity of from 2 to 50 % by weight, based on the preparation.

4. Hair treatment preparations as claimed in claim 3, characterized in that the surface-active phosphonic acids or their water-soluble salts are present in a quantity of from 0.1 to 2.0 % by weight while a mixture of an alkyl polyglycol ether sulfate alkali salt containing 10 to 16 C atoms in the alkyl group and 2 to 4 glycol ether groups and an alkyl polyglycol ether sulfosuccinic acid monoester alkali salt containing 10 to 16 C atoms in the alkyl group and 2 to 4 glycol ether groups is present as the anionic surfactant in a quantity of 2 to 50 % by weight, based on the hair treatment preparation.

## Revendications

1. Compositions pour le traitement des cheveux ayant un additif qui améliore l'aptitude au peignage humide des cheveux, caractérisées en ce que comme additif, au moins un acide phosphonique tensioactif de formule générale (I)

$$R^1—CH_2—PO_3H_2$$

dans laquelle $R^1$ est un groupe alcoyle linéaire ayant de 7 à 13 atomes de carbone, ou un sel de celui-ci est contenu.

2. Compositions pour le traitement des cheveux selon la revendication 1, caractérisées en ce que de l'acide octyl-1 phosphonique, de l'acide décyl-1 phosphonique ou de l'acide dodécyl-1 phosphonique ou un de leurs sels, est contenu.

3. Compositions pour le traitement des cheveux selon les revendications 1 et 2, sous la forme d'un shampooing, caractérisées en ce que les acides phosphoniques tensio-actifs ou leurs sels solubles dans l'eau sont contenus en quantité de 0,05-10 % en poids, ainsi que des agents tensio-actifs en quantité de 2 à 50 % en poids de la composition.

4. Compositions pour le traitement des cheveux selon la revendication 3, caractérisées en ce que les acides phosphoniques tensio-actifs ou leurs sels hydrosolubles sont contenus en quantité de 0,1 à 2 % en poids ainsi que comme agents tensio-actifs anioniques, un mélange à base d'alcoyl polyglycoléther sulfate de métal alcalin ayant 10 à 16 atomes de C dans le radical alcoyle et de 2 à 4 groupes glycoléthers, et de sel de métal alcalin de monoester d'acide alcoylpolyglycoléther sulfosuccinique ayant 10 à 16 atomes de C dans le radical alcoyle et 2 à 4 groupes glycoléther en quantité de 2 à 50 % en poids de la composition pour le traitement des cheveux.